(19) Europäisches Patentamt
European Patent Office
Office européen des brevets

(11) Veröffentlichungsnummer : **0 033 830**
**B1**

(12) # EUROPÄISCHE PATENTSCHRIFT

(45) Veröffentlichungstag der Patentschrift : **03.11.82**

(51) Int. Cl.³ : **C 07 D487/08** // (C07D487/08, 249/00, 229/00)

(21) Anmeldenummer : **81100064.5**

(22) Anmeldetag : **08.01.81**

(54) **Verfahren zur Herstellung polycyclischer stickstoffhaltiger Verbindungen.**

(30) Priorität : **17.01.80 DE 3001580**

(43) Veröffentlichungstag der Anmeldung : **19.08.81 (Patentblatt 81/33)**

(45) Bekanntmachung des Hinweises auf die Patenterteilung : **03.11.82 Patentblatt 82/44**

(84) Benannte Vertragsstaaten : **AT BE CH DE FR GB IT LI NL SE**

(56) Entgegenhaltungen :
**EP A 0 001 271**
**DE A 2 615 878**

**JOURNAL OF THE AMERICAN CHEMICAL SOCIETY, Band 91, 1969, Easton N. RIEBER et al. « Delta 1-1,2-Diazetines » Seiten 5 668 bis 5 669**

(73) Patentinhaber : **BASF Aktiengesellschaft**
**Carl-Bosch-Strasse 38**
**D-6700 Ludwigshafen (DE)**

(72) Erfinder : **Rieber, Norbert, Dr.**
**Liebfrauenstrasse 1C**
**D-6800 Mannheim (DE)**
Erfinder : **Boehm, Heinrich, Dr.**
**Keplerweg 2**
**D-6701 Neuhofen (DE)**
Erfinder : **Platz, Rolf, Dr.**
**Hansastrasse 5**
**D-6800 Mannheim (DE)**
Erfinder : **Fuchs, Werner, Dr.**
**Muenchbuschweg 30B**
**D-6700 Ludwigshafen (DE)**

**EP 0 033 830 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Verfahren zur Herstellung polycyclischer stickstoffhaltiger Verbindungen

Die vorliegende Erfindung betrifft ein Verfahren zur Herstellung von polycyclischen stickstoffhaltigen Verbindungen durch Umsetzung der entsprechenden Dialkoxycarbonylverbindungen mit Alkalilauge oder Calciumhydroxid und Oxydation.

Aus der DE-A-2 615 878 sind polycyclische stickstoffhaltige Verbindungen, ihre Herstellung und Verwendung bekannt. Darunter sind auch Verbindungen mit den Formeln I und II,

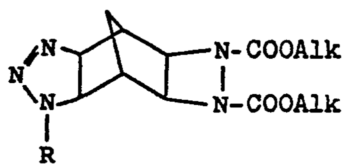

(I)

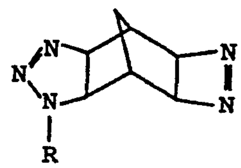

(II)

wobei Alk einen Alkyrest von 1 bis 4 Kohlenstoffatomen und R Wasserstoff oder einen cyclischen, acyclischen, verzweigten oder unverzweigten Alkyl-, Alkenyl- oder Alkinylrest mit 1-30 Kohlenstoffatomen,

einen Phenyl-, Naphthyl- oder einen höherkondensierten Aromatenrest,

einen heterocyclischen Rest mit einem oder mehreren Heteroatomen (O, N, S) oder

einen Aralkylrest, wobei der Aromat auch durch einen Heterocyclus ersetzt sein kann,

bedeutet und die oben angeführten Reste außer Wasserstoff ein- oder mehrfach substituiert sein können.

Diese Verbindungen werden in bekannter Weise nach dem folgenden Schema durch Umsetzung von Alkyl-, Aryl-, Aralkyl-, Silyl- oder heterocyclischen Aziden der Formel R—$N_3$ mit den Verbindungen III oder IV hergestellt, wobei R die oben genannten Bedeutungen hat.

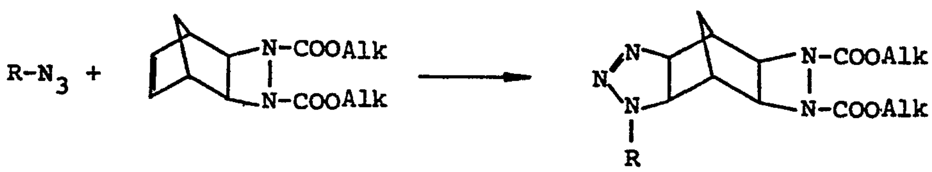

(III) (I)

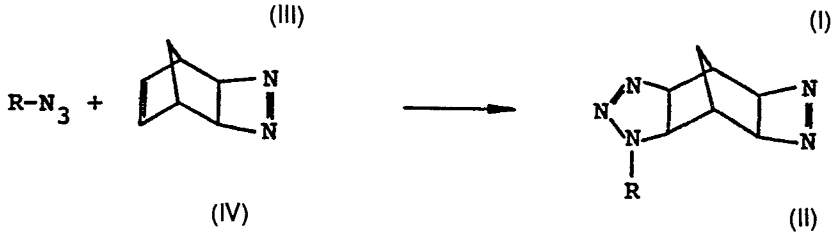

(IV) (II)

Die Verbindung IV erhält man, nach JACS *91*, 5 668 (1969) durch Verseifung und Decarboxylierung von III (Alk = $CH_3$) zum Hydrazin-Derivat, Umwandlung des Hydrazin-Derivates mit $CuCl_2$ in einen $Cu^I$-Komplex von IV, Freisetzung von IV mit wäßriger Natronlauge und Extraktion aus der Natronlaugephase mit organischen Lösungsmitteln in einer Gesamtausbeute von 36 % nach folgenchem Schema.

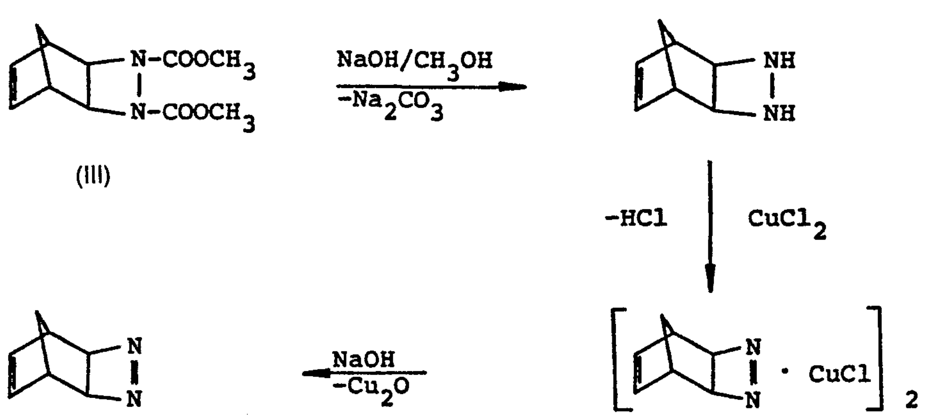


(III)

(IV)

Im Vergleich zur Synthese der Wirkstoffe I ist die Herstellung der Wirkstoffe II nach dem oben angeführten Schema sehr aufwendig, da zuerst die Verbindung III

— unter Einsatz des teuren $CuCl_2$
— über den zu isolierenden Kupferkomplex
— in einer Ausbeute von nur 36 %

in die Verbindung IV umgewandelt werden muß und diese dann mit R—$N_3$ umgesetzt wird.

Aufgabe der vorliegenden Erfindung war die Entwicklung eines einfachen und billigen Verfahrens zur Herstellung der Wirkstoffe II mit hohen Ausbeuten.

Es wurde gefunden, daß bei der Herstellung der Verbindungen der Formel

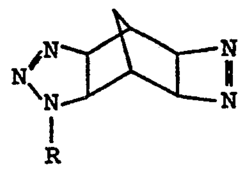

in der

R Wasserstoff oder einen cyclischen, acyclischen, verzweigten oder unwerzweigten Alkyl-, Alkenyl- oder Alkinylrest mit 1 bis 30 Kohlenstoffatomen.
einen Phenyl-, Naphthyl- oder einen höher kondensierten Aromatenrest,
einen heterocyclischen Rest mit einem oder mehreren Heteroatomen (O, N, S) oder
einen Aralkylrest, wobei der Aromat auch durch einen Heterocyclus ersetzt sein kann,

bedeutet und die oben angeführten Reste außer Wasserstoff ein- oder mehrfach substituiert sein können, eine wesentliche Verbesserung erzielt wird, wenn man eine Verbindung der Formel

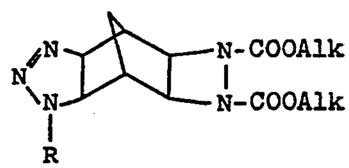

in der

R und Alk die oben genannten Bedeutungen haben, mit einer wäßrigen oder methanolischen Lösung von Alkalihydroxid oder Calciumhydroxid umsetzt und danach mit einer wäßrigen Lösung von Natriumhypochlorit oder Wasserstoffperoxyd oxydiert. Alkalihydroxid ist beispielsweise Natriumhydroxyd oder Kaliumhydroxid oder ihre Mischung.

Das Verfahren kann beispielsweise wie folgt ausgeführt werden.

1. Umsetzung der Azide R—$N_3$ mit der Verbindung III zu den Wirkstoffen I wie in der DE-OS 2 615 878 beschrieben.

2. Überführung der Wirkstoffe I durch Verseifung, Decarboxylierung und Oxydation ohne Isolierung der einzelnen Zwischenstufen, unter Vermeidung der Verwendung des teuren $CuCl_2$, auf einfache Weise mit hoher Ausbeute in die Wirkstoffe II.

Im einzelnen wird das erfindungsgemäße Verfahren beispielsweise so durchgeführt, daß die Wirkstoffe I mit wäßriger oder methanolischer Alkalilauge bei 20-100 °C, vorzugsweise 40-80 °C verseift und decarboxyliert und die intermediär entstehenden Hydrazinderivate ohne Isolierung durch Zugabe von wäßriger NaOCl- oder $H_2O_2$-Lösung zum Reaktionsgemisch bei 20-100 °C, vorzugsweise bei 40 bis 80°, zu den Wirkstoffen II in hohen Ausbeuten oxydiert werden.

Dieser neue Syntheseweg kann bei allen in der DE-A-2 615 878 aufgeführten Wirkstoffen vom Typ II mit allen gegen alkalische NaOCl- oder $H_2O_2$-Lösung stabilen Resten R, in der obigen DE-A als $R^1$ bezeichnet, angewandt werden.

Die Umsetzung der Verbindung III mit dem Azid R—$N_3$ wird beispielsweise mit äquimolaren Mengen der Ausgangsprodukte oder mit einem Überschuß an R—$N_3$ durchgeführt.

Für die Umsetzung der Verbindung I mit der Lösung von Alkalihydroxyd oder Calciumhydroxid werden beispielsweise, abhängig von der Löslichkeit des Hydroxyds, Lösungen des Hydroxyd in Wasser oder Methanol in Konzentrationen von 1 bis 500 g Hydroxid in einem Liter der Lösung verwendet, wobei je Mol der Verbindung I 4 bis 8 Mole Hydroxid verwendet werden.

Für die Oxydation werden wäßrige Lösungen von Natriumhypochlorit oder Wasserstoffsperoxyd verwendet, die je Liter Lösung 1 bis 500 g des Oxydationsmittels enthalten. Für die Oxydation werden je Mol der Verbindung I 1 bis 10 Mole des Oxydationsmittels verwendet.

Für die Synthese des Wirkstoffes der Formel II mit dem Rest R = p-Chlorphenyl wird das erfindungsgemäße Verfahren im folgenden im Vergleich mit dem bekannten Verfahren beschrieben.

Gemäß DE-A-2 615 878 (Beispiel 1) führt die Umsetzung von 92 Teilen (Gewichtsteilen) p-Chlorphenylazid mit 72 Teilen der Verbindung IV in einer Ausbeute von 90 % zu 148 Teilen des Wirkstoffes I (R = p-Chlorphenyl).

Zur Herstellung von 72 Teilen der Verbindung IV auf dem in der DE-A-2 615 878 und in JACS *91*, 5 668 (1969) beschriebenen Syntheseweg werden bei 36 % Ausbeute an der Verbindung IV 396 Teile der Verbindung III benötigt.

Stellt man dieselbe Wirkstoffmenge nach dem erfindungsgemäßen Verfahren her, so sind als Ausgangsprodukte nur 143 Teile der Verbindung III und 92 Teile p-Chlorphenylazid erforderlich. Die Umsetzung von p-Chlorphenylazid mit III zum Wirkstoff I entsprechend Beispiel 2 der DE-A-2 615 878 verläuft dabei mit einer Ausbeute von 93 % und die Umwandlung des Wirkstoffs I in den Wirkstoff II nach dem erfindungsgemäßen Verfahren mit einer Ausbeute von 97 %.

Beispiel 1

339 Teile des Wirkstoffs I (R = p-Chlorphenyl) werden in 1 110 Teilen 25 %ige (Gew.-%) wäßriger Natronlauge suspendiert und 6 Stunden bei 85 °C gerührt. Anschließend werden bei 60 °C 969 Teile 13 %ige wäßrige NaOCl-Lösung in 2 Stunden zugegeben und 2 Stunden bei 60 °C nachgerührt. Nach Abkühlen auf 20 °C wird das Festprodukt abgesaugt, mit 2 000-3 000 Teilen Wasser gewaschen und getrocknet.

Man erhält 230 Teile ($\hat{=}$ 97 mol-%) Wirkstoff II R = p-Chlorphenyl, Fp. 192 °C Zers.

Beispiel 2

Zu einer Suspension von 300 Teilen des Wirkstoffes I (R = p-Chlorphenyl) in 1 000 Teilen Methanol gibt man innerhalb 15 Minuten bei 68 °C unter Rühren eine Lösung von 123 Teilen NaOH in 1 100 Teilen Methanol. Nach der Zugabe rührt man noch 75 Minuten bei 68 °C und fügt dann innerhalb von 15 Minuten 260 Teile 30 %ige wäßrige $H_2O_2$-Lösung zu. Anschließend wird noch 1 Stunde bei 68 °C nachgerührt.

Danach destilliert man 1 600 Teile Methanol im Vakuum bei einer Temperatur der Mischung von bis zu 65 °C ab und gibt innerhalb 15 Minuten 2 000 Teile VE(voll entsalztes)-Wasser unter Rühren zu.

Das ausgefallene Produkt wird abgesaugt, zweimal mit 500 Teilen VE-Wasser gewaschen und getrocknet.

Man erhält 199 Teile des Wirkstoffes II (R = p-Chlorphenyl) ( = 95 mol-%, Fp. 193° Zers.).

Nach dem erfindungsgemäßen Verfahren können die in der DE-A-26 15 878 beschriebenen Verbindungen hergestellt werden, die der Formel II entsprechen.

**Ansprüche**

1. Verfahren zur Herstellung polycyclischer stickstoffhaltiger Verbindungen der Formel

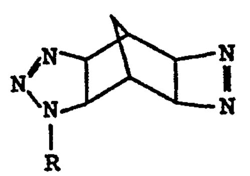

in der

R Wasserstoff oder

einen cyclischen, acyclischen, verzweigten oder unwerzweigten Alkyl-, Alkenyl- oder Alkinylrest mit 1 bis 30 Kohlenstoffatomen,

einen Phenyl-, Naphthyl- oder einen höherkondensierten Aromatenrest,

einen heterocyclischen Rest mit einem oder mehreren Heteroatomen (O, N, S) oder

einen Aralkylrest, wobei der Aromat auch durch einen Heterocyclus ersetzt sein kann,

bedeutet und die oben agenführten Reste außer Wasserstoff ein- oder mehrfach substituiert sein können, dadurch gekennzeichnet, daß man eine Verbindung der Formel

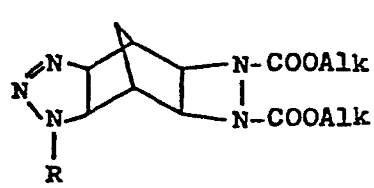

in der

Alk einen Alkyrest mit 1 bis 4 Kohlenstoffatomen bedeutet und R die oben genannten Bedeutungen

hat, mit einer wäßrigen oder methanolischen Lösung von Alkalihydroxyd oder Calciumhydroxid umsetz und danach mit einer wäßrigen Lösung von Natriumhypochlorit oder Wasserstoffperoxyd oxidiert.

2. Verfahren nach Anspruch 1, dadurch gekennzeichnet, daß man die Umsetzung mit Alkalilauge und die Oxydation bei einer Temperatur von 20 bis 100 °C vornimmt.

3. Verfahren nach Anspruch 1, dadurch gekennzeichnet, daß man die Umsetzung mit Alkalilauge und die Oxydation nacheinander ohne Isolierung einer Zwischenverbindung durchführt.

4. Verfahren nach Anspruch 1, dadurch gekennzeichnet, daß man die Umsetzung und die Oxydation in demselben Gefäß vornimmt.

5. Verfahren nach Anspruch 1, dadurch gekennzeichnet, daß man als Ausgangsprodukt für das Verfahren eine Verbindung der Formel

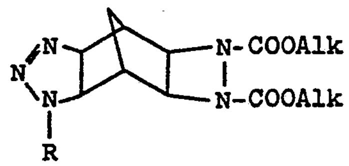

verwendet, die durch Umsetzung einer Verbindung der Formel

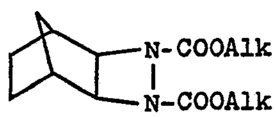

mit einem Azid der Formel $R—N_3$ hergestellt wurde, wobei R und Alk die im Anspruch 1 genannten Bedeutungen haben.

**Claims**

1. A process for the preparation of a polycyclic nitrogen-containing compound of the formula

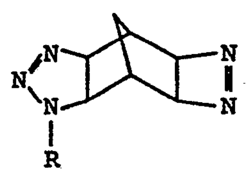

where

R is hydrogen or
is cyclic, acyclic, branched or unbranched alkyl, alkenyl or alkynyl of 1-30 carbon atoms,
phenyl, naphthyl or a more highly condensed aromatic radical,
a heterocyclic radical containing one or more heteroatoms (O, N or S) or
aralkyl in which the aromatic moiety may also be replaced by a heterocyclic ring,

and the above radicals, other than hydrogen, may be monosubstituted or polysubstituted, characterized in that a compound of the formula

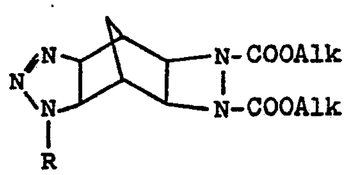

where

Alk is alkyl of 1 to 4 carbon atoms and R has the above meanings, is reacted with an aqueous or methanolic solution of an alkali metal hydroxide or of calcium hydroxide, and the product is then oxidized with an aqueous solution of sodium hypochlorite or hydrogen peroxide.

2. A process as claimed in claim 1, characterized in that the reaction with the alkali metal hydroxide solution, and the oxidation, are carried out at from 20 to 100 °C.

3. A process as claimed in claim 1, characterized in that the reaction with the alkali metal hydroxide solution and the oxidation are carried out successively, without isolating an intermediate compound.

4. A process as claimed in claim 1, characterized in that the reaction and the oxidation are carried out in the same vessel.

5. A process as claimed in claim 1, characterized in that the starting material used for the process is a compound of the formula

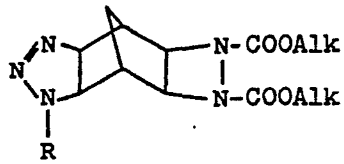

which has been prepared by reacting a compound of the formula

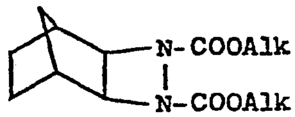

with an azide of the formula R—$N_3$, R and Alk having the meanings given in claim 1.

**Revendications**

1. Procédé de préparation de composés azotés polycycliques de la formule

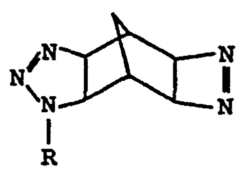

dans laquelle

R peut désigner

un atome d'hydrogène,

un groupe alcoyle, alcényle ou alcynyle cyclique ou acyclique en $C_1$ à $C_{30}$, ramifié ou non,

un groupe phényle, naphtyle ou un groupe aromatique plus fortement condensé,

un groupe hétérocyclique avec un ou plusieurs hétéroatomes (O, N, S),

un groupe aralcoyle, dont la fraction aromatique peut être remplacée par un groupe hétérocyclique, ces groupes (à part l'hydrogène) pouvant être mono- ou poly-substitués, caractérisé en ce que l'on fait réagir un composé de la formule

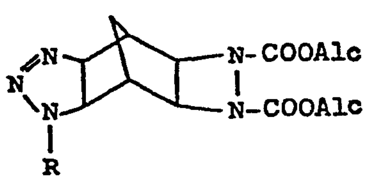

dans laquelle

Alc désigne un radical alcoyle en $C_1$ à $C_4$ et R possède les significations définies ci-dessus, avec un hydroxyde de métal alcalin ou l'hydroxyde de calcium en solution aqueuse ou méthanolique, puis on oxyde à l'aide d'hypochlorite de sodium ou de peroxyde d'hydrogène en solution aqueuse.

2. Procédé suivant la revendication 1, caractérisé en ce que la réaction avec la lessive alcaline et l'oxydation sont réalisées à une température entre 20 et 100 °C.

3. Procédé suivant la revendication 1, caractérisé en ce que la réaction avec la lessive alcaline et l'oxydation sont réalisées successivement et sans isoler un composé intermédiaire.

4. Procédé suivant la revendication 1, caractérisé en ce que la réaction et l'oxydation sont réalisées dans le même réacteur.

5. Procédé suivant la revendication 1, caractérisé en ce que l'on emploie comme produit de départ pour le procédé un composé de la formule

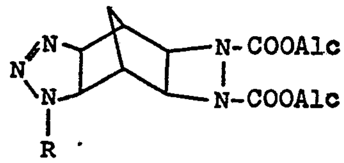

dans laquelle R et Alc possèdent les significations définies dans la revendication 1, obtenu par la réaction d'un composé de la formule

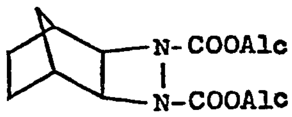

avec un azide de la formule $R—N_3$.